# EUROPEAN PATENT APPLICATION

(11) **EP 4 763 263 A1**
(43) Date of publication of application: **24.06.2026**
(21) Application number: 24853458.8
(22) Date of filing: 12.07.2024
(51) Int. Cl.: A61M 25/10

(54) **ULTRASOUND BALLOON CATHETER**

(30) Priority: 17.08.2023 US 202363533207 P
(71) Applicant: Yeh, Chih-Kuang, Hsinchu City Taiwan 30013 (TW)
(72) Inventor: TSAI, Chieh-Yu, Hsinchu City, Taiwan 30013 (CN); YEH, Chih-Kuang, Hsinchu City, Taiwan 30013 (CN)
(74) Representative: Kuhnen & Wacker Patent- und Rechtsanwaltsbüro PartG mbB
(86) International application number: PCT/CN2024/105085
(87) International publication number: WO 2025/036046

(57) **Abstract**

Provided is an ultrasonic balloon catheter including a miniature ultrasonic emission element, which is an ultrasonic balloon catheter configured to be introduced into a cardiovascular system for the treatment of stenosis or obstruction of a heart or peripheral blood vessel. The ultrasonic balloon catheter includes: a catheter hose, which is a flexible hose: a balloon composed of an elastic material and provided with an expansion part for expansion and contraction and necks arranged at both ends of the expansion part respectively, where the balloon is fixedly arranged outside the catheter hose by using the necks, and the expansion part and the necks form an expansion space at an outer wall of the catheter hose, to allow a fluid composition to be injected into the expansion space through an opening in an outer tube wall; and an ultrasonic probe arranged inside the expansion part of the balloon, sleeved outside an inner tube fitting and at the opening in the outer tube wall, and configured to emit an ultrasonic wave to induce a cavitation effect generated by microbubbles in the fluid composition located in the expansion space.

## Description

### TECHNICAL FIELD

The present application relates to an ultrasonic balloon catheter, and in particular, to an ultrasonic balloon catheter that can induce a cavitation effect through an ultrasonic probe capable of emitting an ultrasonic wave to assist in balloon dilation and alleviate vascular obstruction.

### BACKGROUND

Cardiovascular diseases have long been one of the top five causes of death among Chinese people. With the aging population and gradual westernization of diet, the prevalence and mortality rates of cardiovascular diseases have been increasing year by year, and there is a trend of younger patients with cardiovascular diseases. Coronary artery disease (CAD) and peripheral artery disease (PAD) are the most common cardiovascular diseases. The two diseases are mainly due to the deposition and hardening of fat or other substances in the arteries, which form atheromatous plaques or calcifications, resulting in arteriosclerosis and obstruction to normal blood flow. It is conceivable that insufficient or no blood flow in coronary arteries causes heart disease or even leads to heart failure and sudden death. In addition, peripheral arterial disease may cause pain during walking or poor wound healing in limbs. In severe cases, the peripheral arterial disease leads to critical limb ischemia (CLI), resulting in amputation.

Vascular calcification may cause the vascular wall to harden and lose its due elasticity, resulting in poor contraction and relaxation of the blood vessels. Moreover, excessive calcium deposition on the blood vessel wall can be called vascular calcification. Generally, there are many ways of treating vascular stenosis, vascular sclerosis, and atherosclerosis, such as laser or rotational atherectomy. The most common and cheapest method is percutaneous transluminal angioplasty (PTA), commonly known as balloon catheter dilation, which inflates the balloon by using water pressure, to restore a narrowed blood vessel. However, when there are too severe symptoms of sclerosis, it often leads to the failure to inflate the balloon smoothly and rupture, resulting in the risk of surgery.

### SUMMARY

An objective of the present application is to provide an ultrasonic balloon catheter, which is an ultrasonic balloon catheter that can induce a cavitation effect through an ultrasonic probe capable of emitting an ultrasonic wave to assist in balloon dilation, to alleviate arterial vascular obstruction or refractory vascular calcification.

To achieve the foregoing objective, the present application provides an ultrasonic balloon catheter, configured to be introduced into a cardiovascular system for the treatment of stenosis or obstruction of a heart or peripheral blood vessel, including: a catheter hose, which is a flexible hose, further including: an inner tube fitting having an inner tube wall capable of defining a first accommodating space, where the first accommodating space is used to allow a guide wire to pass through; an outer tube fitting sleeved outside the inner tube wall and having an outer tube wall capable of defining a second accommodating space, where the second accommodating space is used to allow a fluid composition with microbubbles to flow in, and the outer tube wall has at least one opening for allowing the fluid composition to flow out; and an electrode tube fitting arranged inside the outer tube fitting and having an electrode tube wall capable of defining a third accommodating space, where the third accommodating space is used to allow at least one electrode lead to pass through; a balloon composed of an elastic material and provided with an expansion part for expansion and contraction and necks arranged at both ends of the expansion part respectively, where the balloon is fixedly arranged outside the catheter hose by using the necks, and the expansion part and the necks form an expansion space at an outer wall of the catheter hose, to allow the fluid composition to be injected into the expansion space through each opening in the outer tube wall; and at least one ultrasonic probe arranged inside the expansion part of the balloon, sleeved outside the inner tube fitting and at each opening in the outer tube wall, and configured to emit an ultrasonic wave to induce a cavitation effect generated by the microbubbles in the fluid composition located in the expansion space.

In an embodiment of the present application, when the catheter hose passes through a vascular network of the cardiovascular system, the catheter hose is fitted with a guide wire path and slides along the guide wire.

In an embodiment of the present application, in the catheter hose, the inner tube fitting and the outer tube fitting are arranged as concentric circles, and an outer diameter of the inner tube fitting is smaller than an inner diameter of the outer tube fitting.

In an embodiment of the present application, the inner tube fitting is made of a polyether polyamide block (PEBAX) copolymer, a polyamide (NYLON), a thermoplastic polyurethane (TPU) or another high-molecular material with high sound transmission properties.

In an embodiment of the present application, the balloon is made of a polyether polyamide block (PEBAX) copolymer, a polyamide (NYLON), a thermoplastic polyurethane (TPU) or another high-molecular material with high sound transmission properties.

In an embodiment of the present application, the balloon has an action length of 20 mm to 80 mm and a diameter of 2 mm to 7 mm.

In an embodiment of the present application, the balloon has a maximum pressure resistance of 10 atm to 15 atm, and is fully inflated at an air pressure of 3 atm to 7 atm.

In an embodiment of the present application, the at least one ultrasonic probe is a tubular ultrasonic probe, and the ultrasonic probe has an inner diameter of 0.5 mm to 1 mm, an outer diameter of 1.5 mm to 2.5 mm, a height of 2 mm to 6 mm, and resonant frequencies of 100 kHz to 5 MHz and 1 MHz to 10 MHz.

The ultrasonic probe is made of a PZT piezoelectric material, and a surface electrode coating is made of gold, silver, platinum or another high-conductivity material.

In an embodiment of the present application, the at least one ultrasonic probe is a sheet-like combined ultrasonic probe, the sheet-like combination may be two or more sheets, and the ultrasonic probe has a length of 0.2 mm to 1.5 mm, a width of 1 mm to 6 mm, a height of 0.5 mm to 6 mm, and a resonant frequency of 1 MHz to 20 MHz.

The ultrasonic probe is made of a PZT piezoelectric material, and a surface electrode coating is made of gold, silver, platinum or another high-conductivity material.

The ultrasonic balloon catheter according to the present application features at least the following: Through the catheter hose structure arranged as concentric circles, the guide wire is effectively used to select a correct path through the vascular network of the cardiovascular system under the push of a user. Once the correct path is established, the catheter hose slides along the guide wire to a vascular obstruction, so that when the balloon is at a correct position, the fluid composition with microbubbles is injected, and then the ultrasonic probe in the balloon generates an ultrasonic wave to assist in balloon dilatation and induce a cavitation effect, to quickly and efficiently alleviate vascular obstruction and refractory vascular calcification by using energy released in the process from bubble formation to rupture.

### BRIEF DESCRIPTION OF DRAWINGS

FIG. 1 is a schematic structural diagram of an ultrasonic balloon catheter according to the present application;
FIG. 2 is a schematic structural diagram of a catheter hose according to Embodiment 1 of the present application;
FIG. 3 is a schematic structural diagram of a catheter hose according to Embodiment 2 of the present application;
FIG. 4 is a schematic structural diagram of a catheter hose according to Embodiment 3 of the present application;
FIG. 5 is a schematic structural diagram of a catheter hose according to Embodiment 4 of the present application;
FIG. 6 is a schematic structural diagram of an ultrasonic probe according to Embodiment 5 of the present application;
FIG. 7 is an input voltage-output sound pressure curve graph generated by the ultrasonic probe according to Embodiment 5 of the present application;
FIG. 8 is a schematic structural diagram of an ultrasonic probe according to Embodiment 6 of the present application;
FIG. 9 is an input voltage-output sound pressure curve graph generated by the ultrasonic probe according to Embodiment 6 of the present application;
FIG. 10 is a schematic diagram of operations on an ultrasonic balloon catheter according to the present application; and
FIG. 11 is a schematic explanatory diagram of a cavitation effect.

Descriptions of reference signs:
1: Ultrasonic balloon catheter;
11: catheter hose;
111: inner tube fitting;
1111: first accommodating space;
112: outer tube fitting;
1121: second accommodating space;
113: electrode tube fitting;
1131: third accommodating space;
1132: electrode lead;
12: balloon;
121: expansion part;
122: neck;
13: ultrasonic probe;
14: blood vessel.

### DESCRIPTION OF EMBODIMENTS

The present application is further explained below with reference to accompanying drawings and specific embodiments, so that a person skilled in the art can better understand the present application and implement the present application. However, the embodiments given are not taken as limitations on the present application.

In the description of the specification, many specific details are provided for readers to have a more complete understanding of the present application. However, the present application may still be implemented while some or all specific details are omitted. In addition, well-known steps or elements are not described in detail to avoid unnecessarily limiting the present application. The same or similar elements in the drawings are denoted by the same or similar symbols. It should be specially noted that the drawings are only for illustrative purposes and do not denote actual sizes or quantities of elements. Some details may not be fully drawn, to simplify the drawings. Detailed explanations are provided below.

The following embodiments are illustrated with reference to the accompanying drawings, to illustrate specific embodiments based on which the present application can be implemented. Directional terms mentioned in the present application, such as "up", "down", "front", "back", "left", "right", "inside", "outside", and "side surface", are only directions with reference to the accompanying drawings. Therefore, the directional terms used are used to illustrate and understand the present application, not to limit the present application. In addition, in the specification, unless explicitly described to the contrary, the term "including" is to be understood as meaning including the element, but not excluding any other elements.

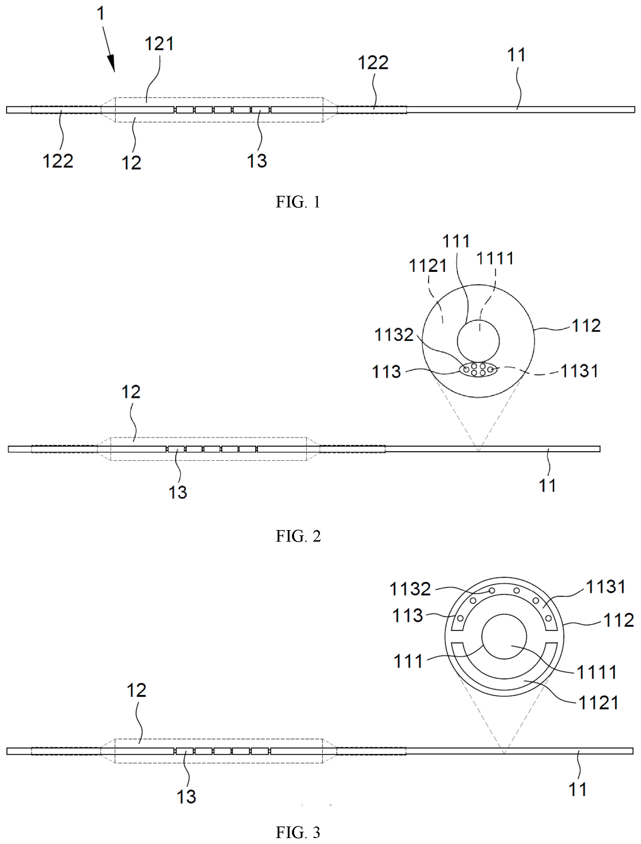

FIG. 1 is a schematic structural diagram of an ultrasonic balloon catheter according to the present application. An ultrasonic balloon catheter 1 shown in FIG. 1 is configured to be introduced into a cardiovascular system for the treatment of stenosis or obstruction of a heart or peripheral blood vessel. The ultrasonic balloon catheter 1 includes: a catheter hose 11, which is a flexible hose, further including: an inner tube fitting having an inner tube wall capable of defining a first accommodating space, where the first accommodating space is used to allow a guide wire to pass through; an outer tube fitting sleeved outside the inner tube wall and having an outer tube wall capable of defining a second accommodating space, where the second accommodating space is used to allow a fluid composition with microbubbles to flow in, and the outer tube wall has at least one opening for allowing the fluid composition to flow out; and an electrode tube fitting arranged inside the outer tube fitting and having an electrode tube wall capable of defining a third accommodating space, where the third accommodating space is used to allow at least one electrode lead to pass through; a balloon 12 composed of an elastic material and provided with an expansion part 121 for expansion and contraction and necks 122 arranged at both ends of the expansion part 121 respectively, where the balloon 12 is fixedly arranged outside the catheter hose 11 by using the necks 122, and the expansion part 121 and the necks 122 form an expansion space at an outer wall of the catheter hose 11, to allow the fluid composition to be injected into the expansion space through each opening in the outer tube wall; and at least one ultrasonic probe 13 arranged inside the expansion part 121 of the balloon 12, sleeved outside the inner tube fitting and at each opening in the outer tube wall, and configured to emit an ultrasonic wave to induce a cavitation effect generated by the microbubbles in the fluid composition located in the expansion space.

In this embodiment, when the catheter hose 11 passes through a vascular network of the cardiovascular system, the catheter hose 11 is fitted with a guide wire path and slides along the guide wire.

The catheter hose 11 is circular, and the balloon 12 can be smoothly guided by the guide wire to an affected part for treatment.

Preferably, the circular structure of the catheter hose 11 enables each component to reach the affected part through a blood vessel without being damaged and operate smoothly.

The caliber of the guide wire may be, but is not limited to, 0.014", 0.018", 0.021" or 0.025".

In this embodiment, in the catheter hose 11, the inner tube fitting and the outer tube fitting are arranged as concentric circles, and an outer diameter of the inner tube fitting is smaller than an inner diameter of the outer tube fitting.

The first accommodating space of the inner tube fitting forms a guide wire cavity.

The guide wire cavity is configured to allow the guide wire to pass through.

The second accommodating space of the outer tube fitting forms a water cavity.

Preferably, the water cavity is configured to allow the fluid composition with microbubbles to flow in.

The third accommodating space of the electrode tube wall is a wire cavity configured to accommodate at least two electrode leads for providing power for the ultrasonic probe 13.

Preferably, the electrode lead includes at least one electrode lead connected to a fire of the ultrasonic probe 13.

Preferably, the electrode lead includes at least one electrode lead connected to an ground of the ultrasonic probe 13.

Preferably, the wire cavity is configured to insulate and cover each electrode lead.

The catheter hose 11 is provided with one or three to five ultrasonic probes 13.

In this embodiment, the inner tube fitting is made of a polyether polyamide block (PEBAX) copolymer, a polyamide (NYLON), a thermoplastic polyurethane (TPU) or another high-molecular material with high sound transmission properties.

In this embodiment, the balloon 12 is made of a polyether polyamide block (PEBAX) copolymer, a polyamide (NYLON), a thermoplastic polyurethane (TPU) or another high-molecular material with high sound transmission properties.

In this embodiment, the balloon 12 has an action length of 20 mm to 80 mm and a diameter of 2 mm to 7 mm.

In this embodiment, the balloon 12 has a maximum pressure resistance of 10 atm to 15 atm, and is fully inflated at an air pressure of 3 atm to 7 atm.

Preferably, the organic polymer material is an ultraviolet curing adhesive (UV adhesive) and is bonded to the catheter hose 11.

In this embodiment, the at least one ultrasonic probe 13 may be a sheet-like combined or tubular ultrasonic probe.

In this embodiment, the at least one ultrasonic probe 13 is a tubular ultrasonic probe, and the ultrasonic probe 13 has an inner diameter of 0.5 mm to 1 mm, an outer diameter of 1.5 mm to 2.5 mm, a height of 2 mm to 6 mm, and resonant frequencies of 100 kHz to 5 MHz and 1 MHz to 10 MHz.

The catheter hose 11 is provided with one or three to five ultrasonic probes 13.

A distance between every two ultrasonic probes 13 is 1 mm to 10 mm.

The ultrasonic probes 13 may be connected in series or in parallel.

Each of the ultrasonic probes 13 may be made of, but limited to, a hard piezoelectric material made of lead zirconate titanate (PTZ).

A surface electrode coating for each ultrasonic probe 13 may be, but is not limited to, an electroless nickel immersion gold, silver or platinum coating.

At least one electrode lead is connected to the fire of the ultrasonic probe 13.

At least one electrode lead is connected to the ground of the ultrasonic probe 13.

A sound transmission rate of a tube wall of the inner tube fitting can reach 70% to 99% at a frequency (100 kHz to 1 MHz) and a distance (0.1 mm to 1 mm).

The sound transmission rate of the tube wall of the inner tube fitting can reach 70% to 99% at a frequency (2 MHz to 6 MHz) and a distance (0.1 mm to 1 mm).

In this embodiment, the at least one ultrasonic probe 13 is a sheet-like combined ultrasonic probe, the sheet-like combination may be two or more sheets, and the ultrasonic probe 13 has a length of 0.2 mm to 1.5 mm, a width of 1 mm to 6 mm, a height of 0.5 mm to 6 mm, and a resonant frequency of 1 MHz to 20 MHz.

The ultrasonic probe 13 is a combination of two, three or more sheets made of a hard piezoelectric material.

The catheter hose 11 is provided with one or three to five ultrasonic probes 13.

Two adjacent ultrasonic probes 13 rotate in an axial direction of the catheter hose 11 by an angle of 135° to 225°.

A distance between every two ultrasonic probes 13 is 1 mm to 10 mm.

The ultrasonic probes 13 may be connected in series or in parallel.

Each of the ultrasonic probes 13 may be made of, but limited to, a hard piezoelectric material made of lead zirconate titanate (PTZ).

A surface electrode coating for each ultrasonic probe 13 may be, but is not limited to, an electroless nickel immersion gold, platinum or silver coating.

At least one electrode lead is connected to the fire of the ultrasonic probe 13.

At least one electrode lead is connected to the ground of the ultrasonic probe 13.

In this embodiment, the cavitation effect is an inertial cavitation effect.

The cavitation effect, also referred to as a cavitation phenomenon, refers to a cavitation effect generated by microbubbles, which is induced when ultrasound waves with certain energy and audio frequency are applied to the microbubbles.

The inertial cavitation effect is a pulse wave generated when microbubbles are extremely compressed and expanded due to strong acoustic energy of ultrasonic waves, so that the microbubbles finally collapse. The forces can remodel a tissue, fat or calcified structure accumulated at an obstructed part.

FIG. 2 is a schematic structural diagram of a catheter hose 11 according to Embodiment 1 of the present application. Referring to FIG. 2, the catheter hose 11 is a flexible hose, and further includes: an inner tube fitting 111 having an inner tube wall capable of defining a first accommodating space 1111, where the first accommodating space 1111 is used to allow a guide wire to pass through; an outer tube fitting 112 sleeved outside the inner tube wall and having an outer tube wall capable of defining a second accommodating space 1121, where the second accommodating space 1121 is used to allow a fluid composition with microbubbles to flow in, and the outer tube wall has at least one opening for allowing the fluid composition to flow out; and an electrode tube fitting 113 arranged inside the outer tube fitting 112 and having an electrode tube wall capable of defining a third accommodating space 1131, where the third accommodating space 1131 is used to allow an electrode lead 1132 to pass through.

In this embodiment, when the catheter hose 11 passes through a vascular network of the cardiovascular system, the catheter hose 11 is fitted with a guide wire path and slides along the guide wire.

The catheter hose 11 is circular, and the balloon 12 can be smoothly guided by the guide wire to an affected part for treatment.

Preferably, the circular structure of the catheter hose 11 enables each component to reach the affected part through a blood vessel without being damaged and operate smoothly.

In this embodiment, the outer tube wall is interrupted at each ultrasonic probe 13, thereby allowing each ultrasonic probe 13 to be in direct contact with the fluid composition in the expansion space of the balloon 12.

In this embodiment, the catheter hose 11 has three cavities, namely, a guide wire cavity of the first accommodating space 1111, a water cavity of the second accommodating space 1121, and a wire cavity of the third accommodating space 1131.

The water cavity and the guide wire cavity are provided as concentric circles.

The guide wire cavity and the water cavity are sequentially provided from inside to outside.

In this embodiment, the wire cavity is provided outside the guide wire cavity.

The wire cavity is configured to allow at least two electrode leads 1132 to pass through, and is configured to insulate and cover each electrode lead 1132.

The wire cavity is bonded to the guide wire cavity by an ultraviolet curing adhesive (UV adhesive) and covers the guide wire cavity.

Preferably, the wire cavity covers the guide wire cavity at an angle of 0° to 180°.

FIG. 3 is a schematic structural diagram of a catheter hose 11 according to Embodiment 2 of the present application. Referring to FIG. 3, the catheter hose 11 is a flexible hose, and further includes: an inner tube fitting 111 having an inner tube wall capable of defining a first accommodating space 1111, where the first accommodating space 1111 is used to allow a guide wire to pass through; an outer tube fitting 112 sleeved outside the inner tube wall and having an outer tube wall capable of defining a second accommodating space 1121, where the second accommodating space 1121 is used to allow a fluid composition with microbubbles to flow in, and the outer tube wall has at least one opening for allowing the fluid composition to flow out; and an electrode tube fitting 113 arranged inside the outer tube fitting 112 and having an electrode tube wall capable of defining a third accommodating space 1131, where the third accommodating space 1131 is used to allow an electrode lead 1132 to pass through.

In this embodiment, when the catheter hose 11 passes through a vascular network of the cardiovascular system, the catheter hose 11 is fitted with a guide wire path and slides along the guide wire.

The catheter hose 11 is circular, and the balloon 12 can be smoothly guided by the guide wire to an affected part for treatment.

Preferably, the circular structure of the catheter hose 11 enables each component to reach the affected part through a blood vessel without being damaged and operate smoothly.

In this embodiment, the outer tube wall is interrupted at each ultrasonic probe 13, thereby allowing each ultrasonic probe 13 to be in direct contact with the fluid composition in the expansion space of the balloon 12.

In this embodiment, the catheter hose 11 has three cavities, namely, a guide wire cavity of the first accommodating space 1111, a water cavity of the second accommodating space 1121, and a wire cavity of the third accommodating space 1131.

In this embodiment, the water cavity is provided outside the guide wire cavity.

Preferably, the water cavity covers the guide wire cavity at an angle of 0° to 180°.

In this embodiment, the wire cavity is provided outside the guide wire cavity.

Preferably, the wire cavity covers the guide wire cavity at an angle of 0° to 180°.

The water cavity and the wire cavity do not coincide.

The wire cavity is configured to allow at least two electrode leads 1132 to pass through, and is configured to insulate and cover each electrode lead 1132.

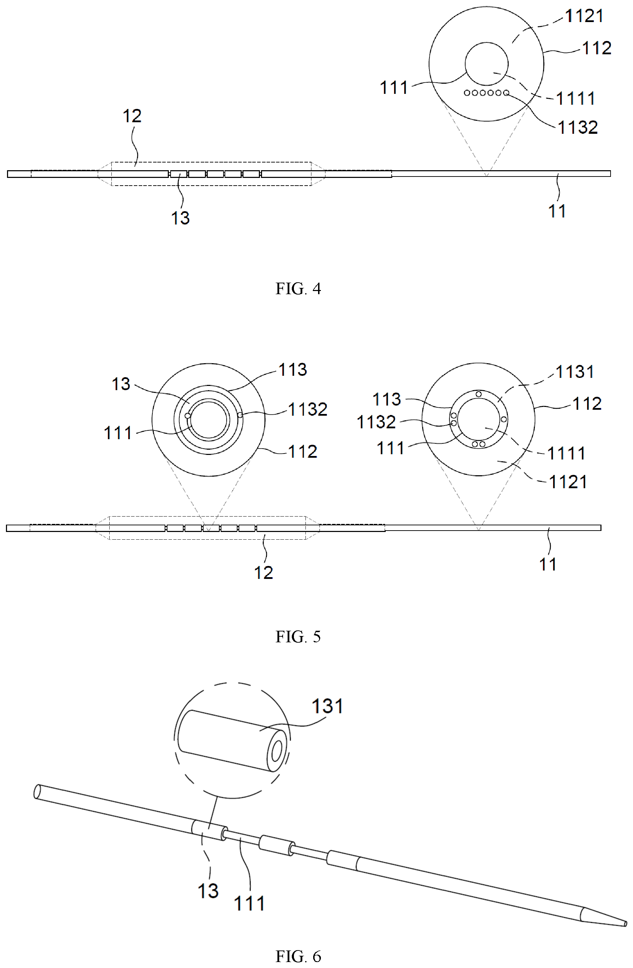

FIG. 4 is a schematic structural diagram of a catheter hose 11 according to Embodiment 3 of the present application. Referring to FIG. 4, the catheter hose 11 is a flexible hose, and further includes: an inner tube fitting 111 having an inner tube wall capable of defining a first accommodating space 1111, where the first accommodating space 1111 is used to allow a guide wire to pass through; an outer tube fitting 112 sleeved outside the inner tube wall and having an outer tube wall capable of defining a second accommodating space 1121, where the second accommodating space 1121 is used to allow a fluid composition with microbubbles to flow in, and the outer tube wall has at least one opening for allowing the fluid composition to flow out and allowing an electrode lead 1132 to pass through.

In this embodiment, when the catheter hose 11 passes through a vascular network of the cardiovascular system, the catheter hose 11 is fitted with a guide wire path and slides along the guide wire.

The catheter hose 11 is circular, and the balloon 12 can be smoothly guided by the guide wire to an affected part for treatment.

Preferably, the circular structure of the catheter hose 11 enables each component to reach the affected part through a blood vessel without being damaged and operate smoothly.

In this embodiment, the outer tube wall is interrupted at each ultrasonic probe 13, thereby allowing each ultrasonic probe 13 to be in direct contact with the fluid composition in the expansion space of the balloon 12.

In this embodiment, the catheter hose 11 has two cavities, namely, a guide wire cavity of the first accommodating space 1111 and a water cavity of the second accommodating space 1121.

The water cavity and the guide wire cavity are provided as concentric circles.

The guide wire cavity and the water cavity are sequentially provided from inside to outside.

In this embodiment, each electrode lead 1132 is arranged outside the guide wire cavity.

FIG. 5 is a schematic structural diagram of a catheter hose 11 according to Embodiment 4 of the present application. Referring to FIG. 5, the catheter hose 11 is a flexible hose, and further includes: an inner tube fitting 111 having an inner tube wall capable of defining a first accommodating space 1111, where the first accommodating space 1111 is used to allow a guide wire to pass through; an outer tube fitting 112 sleeved outside the inner tube wall and having an outer tube wall capable of defining a second accommodating space 1121, where the second accommodating space 1121 is used to allow a fluid composition with microbubbles to flow in, and the outer tube wall has at least one opening for allowing the fluid composition to flow out; and an electrode tube fitting 113 arranged inside the outer tube fitting 112 and having an electrode tube wall capable of defining a third accommodating space 1131, where the third accommodating space 1131 is used to allow an electrode lead 1132 to pass through.

In this embodiment, when the catheter hose 11 passes through a vascular network of the cardiovascular system, the catheter hose 11 is fitted with a guide wire path and slides along the guide wire.

The catheter hose 11 is circular, and the balloon 12 can be smoothly guided by the guide wire to an affected part for treatment.

Preferably, the circular structure of the catheter hose 11 enables each component to reach the affected part through a blood vessel without being damaged and operate smoothly.

In this embodiment, the catheter hose 11 has three cavities, namely, a guide wire cavity of the first accommodating space 1111, a water cavity of the second accommodating space 1121, and a wire cavity of the third accommodating space 1131.

The water cavity, the wire cavity, and the guide wire cavity are provided as concentric circles.

The guide wire cavity, the wire cavity, and the water cavity are sequentially provided from inside to outside.

In this embodiment, the wire cavity is sleeved outside the guide wire cavity.

The wire cavity is configured to allow at least two electrode leads 1132 to pass through, and is configured to insulate and cover each electrode lead 1132.

In this embodiment, each ultrasonic probe 13 is arranged at a gap between the guide wire cavity and the wire cavity.

In this embodiment, the wire cavity is filled with a colloid, where an acoustic impedance value of the colloid is 1.2x106 kg/m2·s to 1.6x106 kg/m2·s.

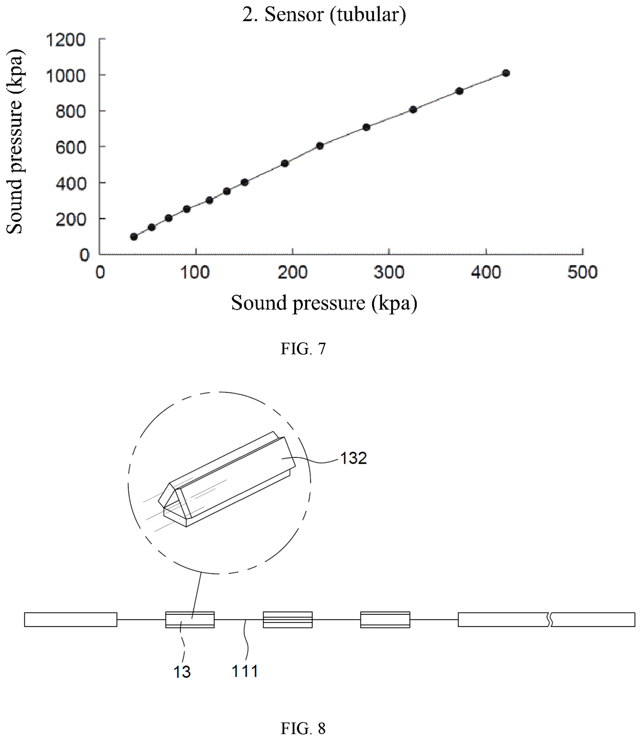

Refer to FIG. 6 and FIG. 7. FIG. 6 is a schematic structural diagram of an ultrasonic probe 13 according to Embodiment 5 of the present application. FIG. 7 is an input voltage-output sound pressure curve graph generated by the ultrasonic probe 13 according to Embodiment 5 of the present application. As shown in FIG. 6, at least one ultrasonic probe 13 is arranged inside the expansion part of the balloon, sleeved outside the inner tube fitting 111 and at each opening in the outer tube wall, and configured to emit an ultrasonic wave to induce a cavitation effect generated by the microbubbles in the fluid composition located in the expansion space.

In this embodiment, the at least one ultrasonic probe 13 is a tubular ultrasonic probe 131, and the ultrasonic probe 13 has an inner diameter of 0.5 mm to 1 mm, an outer diameter of 1.5 mm to 2.5 mm, a height of 2 mm to 6 mm, and resonant frequencies of 100 kHz to 5 MHz and 1 MHz to 10 MHz.

The catheter hose 11 is provided with one or three to five ultrasonic probes 13.

A distance between every two ultrasonic probes 13 is 1 mm to 10 mm.

The ultrasonic probes 13 may be connected in series or in parallel.

Each of the ultrasonic probes 13 may be made of, but limited to, a hard piezoelectric material made of lead zirconate titanate (PTZ).

A surface electrode coating for each ultrasonic probe 13 may be, but is not limited to, an electroless nickel immersion gold, platinum or silver coating.

At least one electrode lead 1132 is connected to the fire of the ultrasonic probe 13.

At least one electrode lead 1132 is connected to the ground of the ultrasonic probe 13.

A sound transmission rate of a tube wall of the inner tube fitting 111 can reach 70% to 99% at a frequency (100 kHz to 1MHz) and a distance (0.1 mm to 1 mm).

The sound transmission rate of the tube wall of the inner tube fitting 111 can reach 70% to 99% at a frequency (2 MHz to 6 MHz) and a distance (0.1 mm to 1 mm).

In this embodiment, as shown in FIG. 7, acoustic properties of the tubular ultrasonic probe 131 are measured by a hydrophone, and it can be found that a greater input voltage indicates a greater output sound pressure and higher energy conversion efficiency of the ultrasonic probe 13.

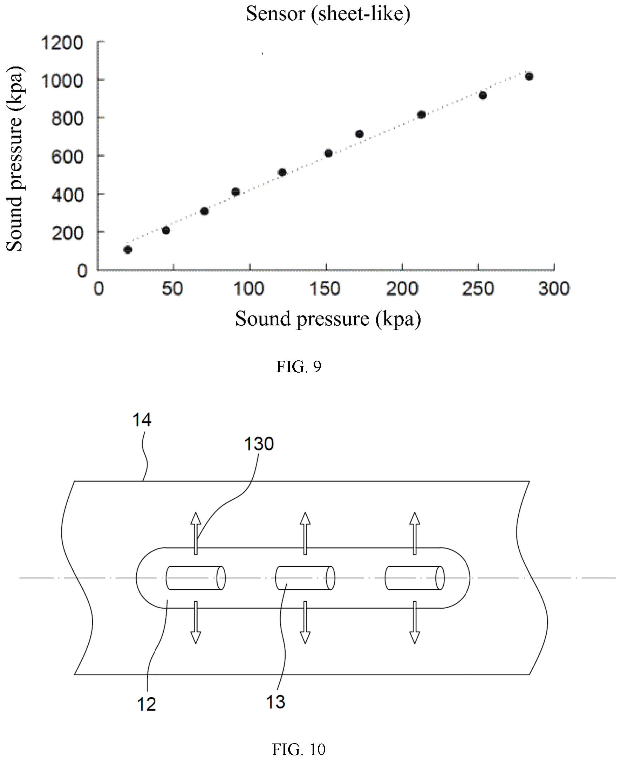

Refer to FIG. 8 and FIG. 9. FIG. 8 is a schematic structural diagram of an ultrasonic probe 13 according to Embodiment 6 of the present application. FIG. 9 is an input voltage-output sound pressure curve graph generated by the ultrasonic probe 13 according to Embodiment 6 of the present application. As shown in FIG. 8, at least one ultrasonic probe 13 is arranged inside the expansion part of the balloon, sleeved outside the inner tube fitting 111 and at each opening in the outer tube wall, and configured to emit an ultrasonic wave to induce a cavitation effect generated by the microbubbles in the fluid composition located in the expansion space.

In this embodiment, the at least one ultrasonic probe 13 is a sheet-like combined ultrasonic probe 132, the sheet-like combined ultrasonic probe 132 may be two or more sheets, and the ultrasonic probe 13 has a length of 0.2 mm to 1.5 mm, a width of 1 mm to 6 mm, a height of 0.5 mm to 6 mm, and a resonant frequency of 1 MHz to 20 MHz.

The ultrasonic probe 13 is a combination of two, three or more sheets made of a hard piezoelectric material.

The catheter hose 11 is provided with one or three to five ultrasonic probes 13.

Two adjacent ultrasonic probes 13 rotate in an axial direction of the catheter hose 11 by an angle of 135° to 225°.

A distance between every two ultrasonic probes 13 is 1 mm to 10 mm.

The ultrasonic probes 13 may be connected in series or in parallel.

Each of the ultrasonic probes 13 may be made of, but limited to, a hard piezoelectric material made of lead zirconate titanate (PTZ).

A surface electrode coating for each ultrasonic probe 13 may be, but is not limited to, a platinum, electroless nickel immersion gold or silver coating.

At least one electrode lead 1132 is connected to the fire of the ultrasonic probe 13.

At least one electrode lead 1132 is connected to the ground of the ultrasonic probe 13.

In this embodiment, as shown in FIG. 9, acoustic properties of the sheet-like combined ultrasonic probe 132 are measured by a hydrophone, and it can be found that a greater input voltage indicates a greater output sound pressure and higher energy conversion efficiency of the ultrasonic probe 13.

FIG. 10 is a schematic diagram of operations on an ultrasonic balloon catheter 1 according to the present application. Referring to FIG. 10, after being introduced into a cardiovascular system, the ultrasonic balloon catheter 1 according to the present application enters a blood vessel 14. When a balloon 12 is at a vascular obstruction, a fluid composition with microbubbles flows into an expansion space in the balloon 12 through an outer tube fitting 112 in the catheter hose 11. In addition, each ultrasonic probe 13 emits an ultrasonic wave 130. An inertia cavitation effect is induced through the ultrasonic waves 130, so that the microbubbles are extremely compressed and expanded due to strong acoustic energy of the ultrasonic waves 130, and the microbubbles finally collapse to generate a pulse wave. In addition, dilation of the balloon 12 and the energy of the ultrasonic waves 130 are used to assist in dilation at the obstruction of the blood vessel to restore smooth blood flow.

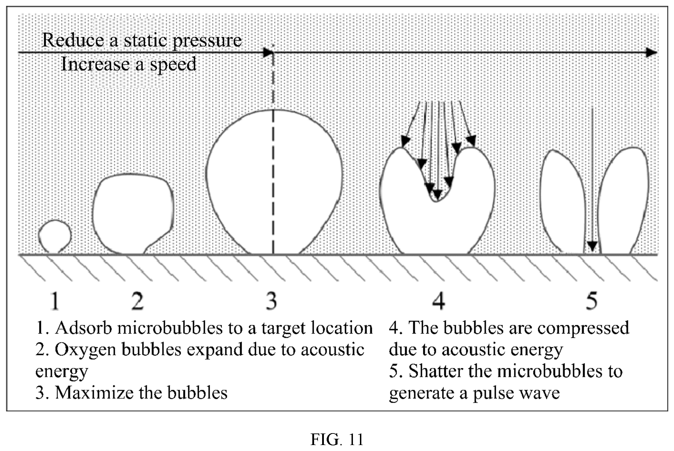

FIG. 11 is a schematic explanatory diagram of a cavitation effect. The cavitation effect, also referred to as a cavitation phenomenon, refers to a cavitation effect generated by microbubbles, which is induced when ultrasound waves with a certain energy and audio frequency are applied to the microbubbles.

In this embodiment, the cavitation effect is an inertial cavitation effect. When the microbubbles are introduced into a blood vessel and adsorbed to a target location, the ultrasonic waves can work. When the microbubbles are extremely compressed and expanded due to strong acoustic energy of the ultrasonic waves, shock waves are generated to shatter the microbubbles, so that the microbubbles finally collapse to generate a pulse wave. The forces can remodel a tissue, fat or calcified structure accumulated at an obstructed part.

To sum up, the ultrasonic balloon catheter according to the present application features at least the following: Through the catheter hose structure arranged as concentric circles, the guide wire is effectively used to select a correct path through the vascular network of the cardiovascular system under the push of a user. Once the correct path is established, the catheter hose slides along the guide wire to a vascular obstruction, so that when the balloon is at a correct position, the fluid composition with microbubbles is injected, and then the ultrasonic probe in the balloon generates an ultrasonic wave to assist in balloon dilatation and induce a cavitation effect, to quickly and effectively alleviate peripheral vascular obstruction and refractory vascular calcification by using energy released in the process from bubble formation to rupture.

The foregoing embodiments are only the preferred embodiments provided to fully illustrate the present application, and the protection scope of the present application is not limited thereto. Any equivalent substitution or transformation made by a person skilled in the art based on the present application falls within the protection scope of the present application. The protection scope of the present application is subject to the claims.

## Claims

1. An ultrasonic balloon catheter, configured to be introduced into a cardiovascular system for the treatment of stenosis or obstruction of a heart or peripheral blood vessel, comprising:
a catheter hose, which is a flexible hose, further comprising:
an inner tube fitting having an inner tube wall capable of defining a first accommodating space, wherein the first accommodating space is used to allow a guide wire to pass through;
an outer tube fitting sleeved outside the inner tube wall and having an outer tube wall capable of defining a second accommodating space, wherein the second accommodating space is used to allow a fluid composition with microbubbles to flow in, and the outer tube wall has at least one opening for allowing the fluid composition to flow out; and
an electrode tube fitting arranged inside the outer tube fitting and having an electrode tube wall capable of defining a third accommodating space, wherein the third accommodating space is used to allow at least one electrode lead to pass through;
a balloon composed of an elastic material and provided with an expansion part for expansion and contraction and necks arranged at both ends of the expansion part respectively, wherein the balloon is fixedly arranged outside the catheter hose by using the necks, and the expansion part and the necks form an expansion space at an outer wall of the catheter hose, to allow the fluid composition to be injected into the expansion space through each opening in the outer tube wall; and
at least one ultrasonic probe arranged inside the expansion part of the balloon, sleeved outside the inner tube fitting and at each opening in the outer tube wall, and configured to emit an ultrasonic wave to induce a cavitation effect generated by the microbubbles in the fluid composition located in the expansion space.

2. The ultrasonic balloon catheter according to claim 1, wherein when the catheter hose passes through a vascular network of the cardiovascular system, the catheter hose is fitted with a guide wire path and slides along the guide wire.

3. The ultrasonic balloon catheter according to claim 1, wherein in the catheter hose, the inner tube fitting and the outer tube fitting are arranged as concentric circles, and an outer diameter of the inner tube fitting is smaller than an inner diameter of the outer tube fitting.

4. The ultrasonic balloon catheter according to claim 1, wherein the inner tube fitting is made of a polyether polyamide block copolymer, a polyamide, a thermoplastic polyurethane or another high-molecular material with high sound transmission properties.

5. The ultrasonic balloon catheter according to claim 1, wherein the balloon is made of a polyether polyamide block copolymer, a polyamide, a thermoplastic polyurethane or another high-molecular material with high sound transmission properties.

6. The ultrasonic balloon catheter according to claim 1, wherein the balloon has an action length of 20 mm to 80 mm and a diameter of 2 mm to 7 mm.

7. The ultrasonic balloon catheter according to claim 1, wherein the balloon has a maximum pressure resistance of 10 atm to 15 atm, and is fully inflated at an air pressure of 3 atm to 7 atm.

8. The ultrasonic balloon catheter according to claim 1, wherein the necks of the balloon are bonded to the catheter hose by using an ultraviolet curing adhesive.

9. The ultrasonic balloon catheter according to claim 1, wherein the at least one ultrasonic probe is a tubular ultrasonic probe, and the ultrasonic probe has an inner diameter of 0.5 mm to 1 mm, an outer diameter of 1.5 mm to 2.5 mm, a height of 2 mm to 6 mm, and resonant frequencies of 100 kHz to 5 MHz and 1 MHz to 10 MHz.

10. The ultrasonic balloon catheter according to claim 1, wherein the at least one ultrasonic probe is a sheet-like combined ultrasonic probe, the sheet-like combination is two or more sheets, and the ultrasonic probe has a length of 0.2 mm to 1.5 mm, a width of 1 mm to 6 mm, a height of 0.5 mm to 6 mm, and a resonant frequency of 1 MHz to 20 MHz.
